# EUROPÄISCHE PATENTSCHRIFT

(11) **EP 3 265 768 B1**
(45) Veröffentlichungstag und Bekanntmachung des Hinweises auf die Patenterteilung: **30.10.2019**
(21) Anmeldenummer: 16705541.7
(22) Anmeldetag: 22.02.2016
(51) Int. Cl.: A61B 5/103, A61B 5/00, G01L 1/14

(54) **KRAFTSENSOR**
FORCE SENSOR
CAPTEUR DE FORCE

(30) Priorität: 06.03.2015 DE 102015103261
(43) Veröffentlichungstag der Anmeldung: 10.01.2018
(73) Patentinhaber: Seitz, Peter, D-81675 München (DE); Novel GmbH, 81675 München (DE)
(72) Erfinder: SEITZ, Peter, 81675 München (DE); DAHROUJ, Ahmad, 81925 München (DE)
(74) Vertreter: Meissner Bolte Partnerschaft mbB
(86) Internationale Anmeldenummer: PCT/EP2016/053620
(87) Internationale Veröffentlichungsnummer: WO 2016/142156

(56) Entgegenhaltungen:
- WO-A1-2014/068269
- US-A- 3 782 486
- US-A- 3 875 481

## Beschreibung

Die Erfindung betrifft einen (Einzel-)Sensor zur elektrischen Messung einer auf den Sensor wirkenden Kraft, die über eine Messfläche des Sensors ungleichmäßig verteilt ist.

Eine Personenwaage ist geeignet, eine auf sie wirkende Kraft, z.B. eine Kraft entsprechend dem Gewicht eines Benutzers, zu messen, auch wenn diese Kraft ungleichmäßig auf der starren Standfläche verteilt ist. Problematisch wird so eine Messung dann, wenn man eine, von einem Benutzer aufgebrachte Kraft z.B. in einem Schuh messen will. Eine derartige Messung ist bei verschiedenen medizinischen Indikationen notwendig, wobei insbesondere dann, wenn die "überwachte" Person ein bestimmtes Training durchführen soll und einen einfachen Zugang zur aufgebrachten Kraft bekommen muss.

Aus der DE 36 34 855 C1 ist eine kapazitive Messanordnung zur Bestimmung von Kräften oder Drücken bekannt, bei welcher eine Vielzahl von Einzelsensoren über eine Fläche, z.B. eine Einlegesohle in einen Schuh verteilt ist. Die Sensoren bestehen bei der bekannten Anordnung aus Kondensatoren, also aus einer Vielzahl von Kondensatorflächen, die matrixförmig aufgebaut und als Matrix einzeln abgetastet werden. Aus der Summe der Einzel-Kräfte bzw. Einzel-Drücke kann eine Gesamtkraft bzw. ein Gesamtdruck errechnet werden. Hierbei spielt es kaum eine Rolle, ob einzelne Bereiche der gesamten Fläche stärker oder weniger stark belastet werden, da ja die Gesamtfläche in kleinere Teilflächen unterteilt wird, so dass Teil-Kräfte gemessen und aufsummiert werden können. Werden bei dieser Anordnung die wirkenden Kräfte über die Messfläche hinaus verteilt, ist also die Gesamtsensorfläche zu klein, so ist kein korrektes Messergebnis herleitbar.

Dann, wenn ein elastischer Sensor (wie oben erwähnt) benötigt wird, sind die Messergebnisse bei ganzflächigen Sensorelementen, z.B. Kondensatorfolien, extrem ungenau bei ungleichmäßig verteilter Kraft.

Aus der US 3,782,486 und der WO 2014/068269 A1 sind Sensoren zur elektrischen Messung einer auf den Sensor wirkenden Kraft bekannt. Die jeweiligen Sensoren umfassen Kondensatorplatten sowie dazwischen angeordnete Federelemente, wobei die Verformung des Sensors kapazitativ messbar ist. Weiterhin wird auf die US 3,875,481 A verwiesen.

Der Erfindung liegt die Aufgabe zu Grunde, ausgehend von der US 3,782,486 A, einen Sensor der eingangs genannten Art dahingehend aufzuzeigen, dass in einfacher Weise eine korrekte Summen-Kraftmessung herleitbar ist.

Diese Aufgabe wird durch einen Sensor nach Anspruch 1 gelöst. Besondere Verwendungen des Sensors sind in Anspruch 9 genannt.

Überraschenderweise hat es sich gezeigt, dass bei Belastung des Sensors durch Kräfte innerhalb eines vorgegebenen Messbereiches nicht mit einer "über alles" Linearisierung gearbeitet werden muss, wie dies bei steifen Sensoren gemacht wird, bei denen eine gleichmäßige Kräfteverteilung auf alle Bereiche des Sensors stattfindet, gleichgültig, wie die Verteilung der einwirkenden Kräfte aussieht. Je nach dem vorgegebenen Messbereich kann dann die Gesamtanordnung derart getroffen werden, dass die Federkörper auch innerhalb flächenmäßig kleiner Messbereiche eine lineare Charakteristik aufweisen. Dadurch ist es möglich, mittels des Sensors eine Kraftsumme zu messen, und zwar im Wesentlichen unabhängig von der Verteilung der einwirkenden Teilkräfte.

Weiterhin sind die Messfläche und die Gegenfläche samt den Messelementen verformbar und zwar insbesondere elastisch verformbar ausgebildet. Es werden also nicht - wie z. B. bei einer Personenwaage - starre Flächen als Messfläche und Gegenfläche verwendet, sondern elastisch nachgiebige Flächen, so dass beispielsweise eine Einlegesohle in einen Schuh als Sensor ausgebildet werden kann. Vorzugsweise sind hierbei die Messfläche und/oder die Gegenfläche als textiles Material ausgebildet bzw. umfassen ein textiles Material, das insbesondere ein Gewirke oder ein Gewebe sein kann. Derartige Materialien sind an sich bekannt. Besonders bevorzugt ist eine Ausführungsform, bei welcher die Messelemente Kondensatorplatten umfassen, wobei eine Kapazität zwischen den Kondensatorplatten zur Erzeugung des Kraftmesssignals messbar ist. Die hierfür notwendige Technik ist bereits recht ausgereift.

An dieser Stelle sei darauf hingewiesen, dass die Messelemente auch als Induktivitäten oder als Ohm'sche Widerstände (oder Mischungen hiervon) aufgebaut sein können und in der nachfolgenden Beschreibung Kondensatorplatten bzw. Kondensatoren als Messelemente als bevorzugtes Ausführungsbeispiel erläutert werden.

Die Messfläche und/oder die Gegenfläche sind mit den Federkörpern durch Befestigungsmittel verbunden. Diese Befestigungsmittel können in vielfältiger Weise ausgebildet sein. Wichtig ist hierbei jedoch, dass die Messfläche und die Gegenfläche derart mit den Federkörpern verbunden sind, dass die geometrische Gesamtanordnung im Wesentlichen konstant bleibt. Hierbei sind die Befestigungsmittel vorzugsweise derart elastisch ausgebildet, dass die Messfläche und/oder die Gegenfläche relativ zu den Federeinrichtungen elastisch verschiebbar sind. Dies ist insbesondere dann notwendig, wenn Wölbungen der Messfläche bzw. der Gegenfläche auftreten. Derart verschiebbare Befestigungsmittel können beispielsweise elastische Verklebungen sein.

Die Messfläche und die Gegenfläche weisen vorzugsweise zusätzlich zum ersten bzw. zweiten Messelement auf ihren, der Federeinrichtung abgewandten Seiten Abschirmungselemente zur elektrischen Abschirmung der Messelemente auf. Dadurch können auch kleinere Messsignale störungsarm erfasst werden.

Die Federkörper können in verschiedener Weise als geometrische Körper aufgebaut werden. Sie können balkenförmige, pyramidenstumpfförmige oder kegelstumpfförmige Einzelelemente umfassen, wobei unter "pyramidenstumpfförmig" bzw. "kegelstumpfförmig" auch Quader bzw. Zylinder mit sehr geringen Abwinklungen gemeint sind. Diese Einzelelemente sind in regelmäßigen Abständen voneinander angeordnet. Als Material eignet sich hier insbesondere Silikonkautschuk, wobei Shore-Härten von 45-55, insbesondere 47-53 bevorzugt sind. Eine Anpassung an die zu messenden Kräfte kann hier natürlich vorgenommen werden.

Die Federkörper können - wie oben beschrieben - durch eine Verklebung oder aber durch eine Trägerfläche miteinander verbunden sein, von welcher die einzelnen Federkörper entweder einseitig oder nach beiden Seiten (mit der Trägerfläche in der Mitte) hervorstehen. In beiden Fällen ist die Herstellung des Sensors dann besonders einfach und präzise, wenn die Trägerfläche mit den Federkörpern einstückig ausgebildet ist.

Wenn die Messelemente Kondensatorplatten umfassen, ist vorzugsweise eine Kapazitätsmesseinrichtung vorgesehen, die mit den Abschirmungselementen und den Kondensatorplatten verbunden und derart ausgebildet ist, dass Kapazitäten zwischen den Kondensatorplatten und den Abschirmungselementen gemessen werden können. Nachdem - in an sich bekannter Weise - alle Kondensatorplatten und auch die Abschirmungen voneinander durch Dielektrika getrennt sind, kann hieraus ein Messsignal generiert werden, welches im Wesentlichen den temperaturabhängigen Materialeigenschaften der Dielektrika entspricht, also insbesondere der material- und temperaturabhängigen Dielektrizitätskonstante. Mittels dieses Messsignals kann in einer Korrektureinrichtung das Kraftmesssignal hinsichtlich seiner Temperaturabhängigkeit korrigiert werden.

Es sind viele verschiedene Kombinationen aus Form und Material für die Federelemente denkbar, die die genannte Linearisierung ermöglichen. Bei einer Ausführungsform sind die Federkörper aus geometrisch verschieden aufgebauten Federkörpern in Gruppen von Federkörpern zusammengesetzt, die jeweils zueinander verschiedene Federcharakteristiken derart aufweisen, dass diese einander kompensieren und eine Linearisierung der Gesamtfedercharakteristik der Gruppe bewirken.

Ein Sensor der hier gezeigten Art kann vorteilhafterweise für eine Vielzahl von Messaufgaben eingesetzt werden. Insbesondere sind dies solche Messaufgaben, bei denen die Flächen, zwischen welchen die Kräfte auftreten, gekrümmt oder - was noch schwieriger hinsichtlich der auszuwählenden Sensoren ist - im Verlaufe der Messung variabel sind. Insbesondere handelt es sich hierbei um Messungen von Belastungen, welche menschliche Gliedmaßen oder Effektoren eines Roboters auf eine Umgebung, z.B. einen Schuh, eine Prothese, eine Orthese, einen Griff, ein Lenkrad oder ein natürliches oder künstliches Gelenk sowie Werkzeuge oder Werkstücke ausüben. Auch Kräfte von Körperteilen auf Unterlagen, z. B. einem Autositz, einer Matratze, einem Liegestuhl oder einem Reit-Sattel sind hierunter zu verstehen.

Nachfolgend werden bevorzugte Ausführungsformen der Erfindung anhand schematisierter Zeichnungen näher dargestellt. Hierbei zeigen
- Fig. 1: ein Teil-Querschnitt durch eine erste Ausführungsform der Erfindung,
- Fig. 2: eine Darstellung der Anordnung nach Fig. 1 bei Belastung mit einer gleichmäßigen Kraft,
- Fig. 3: eine Darstellung der Belastung einer Anordnung nach Fig. 1 mit einer ungleichmäßigen Kraft,
- Fig. 4: eine Draufsicht auf die Anordnungen nach den Fig. 1-3 unter Fortlassung der Messfläche, wobei die strichpunktierte Linie A-A den Schnitten gemäß Fig. 1-3 entspricht,
- Fig. 5: einen detaillierteren Teil-Schnitt einer weiteren Ausführungsform der Erfindung,
- Fig. 6: einen Schnitt ähnlich dem nach Fig. 1, jedoch durch eine Anordnung gemäß Fig. 5,
- Fig. 7: einen Schnitt entsprechend dem nach den Fig. 1 und 6 durch eine weitere Ausführungsform der Erfindung,
- Fig. 8: eine Draufsicht durch eine weitere Ausführungsform der Erfindung,
- Fig. 9: einen Schnitt entlang der Linie IX-IX aus Fig. 8,
- Fig. 10: eine Draufsicht auf eine weitere Ausführungsform der Erfindung,
- Fig. 11: einen Schnitt entlang der Linie XI-XI aus Fig. 10,
- Fig. 12: eine Draufsicht auf eine weitere Ausführungsform der Erfindung,
- Fig. 13: einen Schnitt entlang der Linie XIII-XIII aus Fig. 12,
- Fig. 14: ein beispielhaftes Diagramm zur Darstellung eines linearen Bereiches der Federeinrichtung,
- Fig. 15: eine prinzipielle Querschnittsdarstellung von Gruppen von Federkörpern, und
- Fig. 16: ein Diagramm entsprechend dem nach Fig. 15 zur Erläuterung der Federcharakteristik einer Anordnung nach Fig. 15.

In der nachfolgenden Beschreibung werden für gleiche und gleichwirkende Teile dieselben Bezugsziffern verwendet.

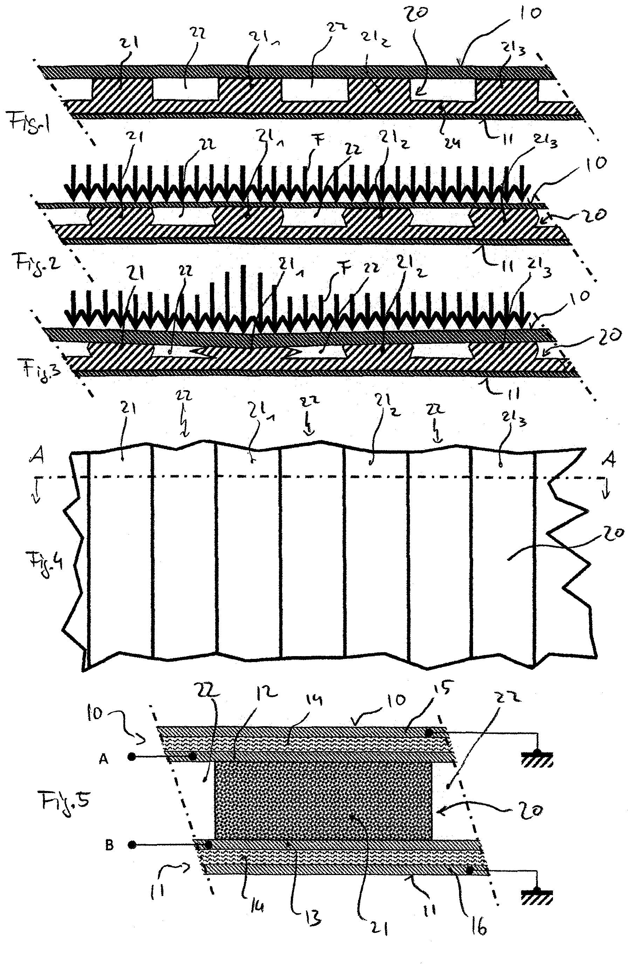

Gemäß Fig. 1 umfasst der Sensor eine Messfläche 10 und eine Gegenfläche 11, wobei zwischen den Messflächen 10 und 11 Federeinrichtungen 20 vorgesehen sind. Diese Federeinrichtungen 20 umfassen Federkörper 21, 21₁, 21₂ und 21₃, die über eine Trägerfläche 24 miteinander verbunden sind. Die Federkörper 21... sind einstückig mit der Trägerfläche 24 aus Silikonkautschuk geformt.

Wenn nun eine Kraft F, wie dies in Fig. 2 gezeigt ist, auf die Messfläche 10 wirkt, so verformen sich die Federkörper 21₁ bis 21₃, dehnen sich also in Lücken 22 hinein, welche zwischen den Körpern 21₁ bis 21₃ vorgesehen sind.

Wenn die wirkende Kraft F ungleichmäßig über die Messfläche 10 verteilt ist, wie dies in Fig. 3 gezeigt ist, verformen sich einzelnen Federkörper 21₁ stärker als die benachbarten Federkörper 21 und 21₂, wobei gleichzeitig die Messfläche 10 verformt wird. Es ist also wichtig, dass die Messfläche 10 und im Allgemeinen auch die Gegenfläche 11 elastisch verformbar sind.

Die in den Fig. 1-3 gezeigte Federeinrichtung 20 besteht - wie in Fig. 4 gezeigt - aus Balken. Dies ist eine besonders einfache "Grundform".

Bei der in Fig. 5 gezeigten Anordnung ist ein einzelner Federkörper 21 vorgesehen, der im Wesentlichen als Quader ausgebildet ist. Dieser Federkörper 21 ist von beiden Seiten mit Messelementen bzw. Elektroden 12 und 13 bedeckt. Zwischen den Elektroden 12 und 13 und außenliegenden Abschirmungselementen 15, 16 ist ein Dielektrikum 14 vorgesehen. Die Gesamtanordnung, also die Messflächen 10, 11, bestehend aus den Messelementen 12, 13, den Abschirmungselementen 15, 16 und dem Dielektrikum 14, ist elastisch verformbar ausgebildet, damit eine Verformung so wie in Fig. 3 gezeigt stattfinden kann.

Nachdem die Kapazität der zwischen den Messelementen bzw. Elektroden 12, 13 angeordneten Federkörper 21 hinsichtlich ihrer Dielektrizitätskonstante temperaturabhängig ist, ist es von Vorteil, wenn diese Temperaturabhängigkeit kompensiert werden kann. Hierzu werden im Multiplex-Betrieb die Messpunkte A und B parallelgeschaltet und die Kapazität zwischen den (parallelgeschalteten) Elektroden 12, 13 und den Abschirmungselementen 15, 16 wechselweise zur Kapazität zwischen den Messpunkten A und B, also zwischen den Elektroden 12 und 13 gemessen. Aus diesen Messungen kann ein Korrektursignal ermittelt werden, welches die Kapazität zwischen den Messelementen bzw. Elektroden 12, 13 im Wesentlichen unabhängig von der Temperatur der Gesamtanordnung machen kann. Die hierfür notwendige elektrische Schaltung ist nicht gesondert aufgezeigt, da sie im Prinzip dem hier tätigen Fachmann geläufig ist.

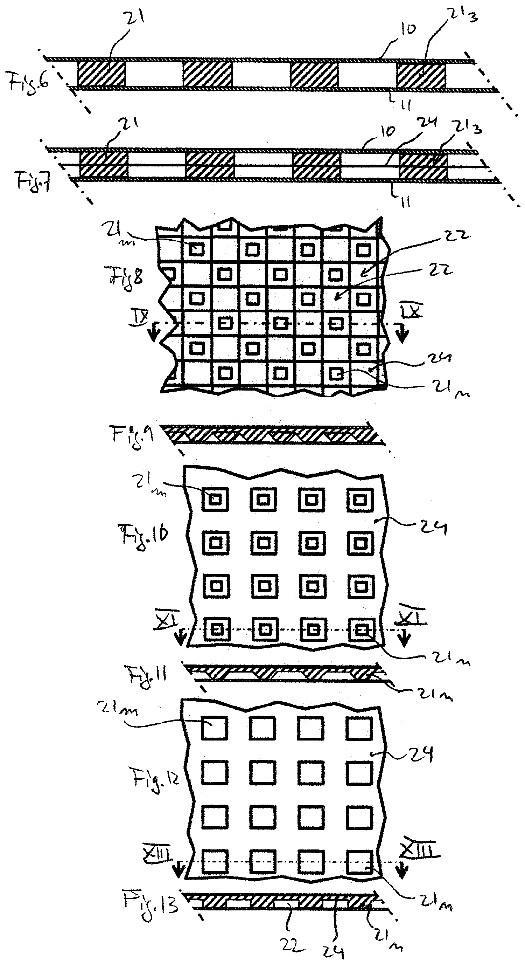

In Fig. 6 ist nochmals eine Anordnung entsprechend der nach Fig. 5 gezeigt. Hierbei sind die Federkörper 21 bis 21₃ direkt mit der Messfläche 10 bzw. der Gegenfläche 11 verbunden, was z. B. durch Kleben geschehen kann. Wenn die Klebeschicht elastisch ist, so ist dies dann von Vorteil, wenn eine Wölbung bzw. Durchbiegung der Gesamtanordnung stattfindet.

Bei der Anordnung nach Fig. 7 sind die Federkörper 21 bis 21₃ beidseitig einer Trägerfläche 24 angeordnet, so dass eine sehr symmetrische Anordnung entsteht.

Bei der Anordnung nach Fig. 8 sind die Federkörper 21ₘ bis 21ₙ pyramidenstumpfförmig ausgebildet, wie dies in Fig. 9 gezeigt ist. Hierbei ist die Anordnung derart getroffen, dass ein seitliches Ausweichen der Federkörper 21ₘ bis 21ₙ möglich ist.

Die Anordnung nach den Fig. 10 und 11 unterscheidet sich von der nach Fig. 8 durch ein größeres Raster an Federkörpern 21ₘ bis 21ₙ.

Die Anordnung nach den Fig. 12 und 13 entspricht der nach Fig. 10, wobei jedoch der Winkel der Pyramidenstümpfe zur Trägerfläche 24 im Wesentlichen 90° beträgt.

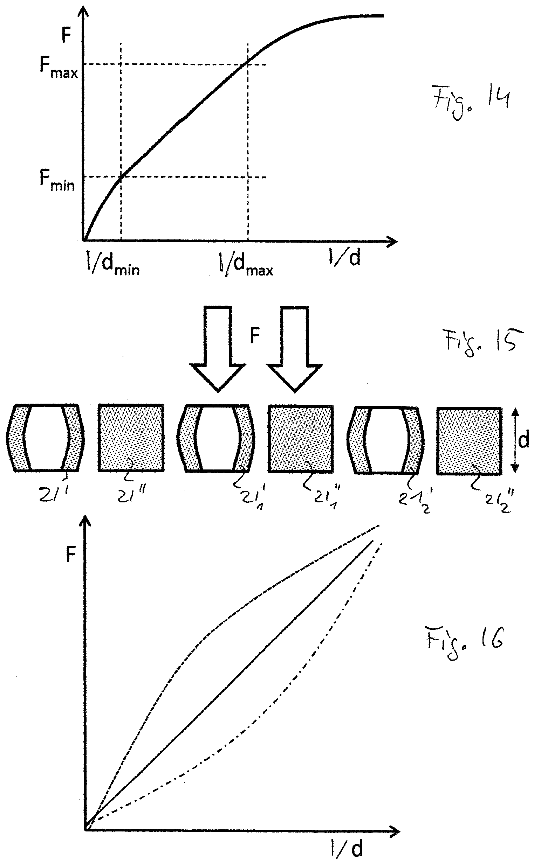

Die eingangs erwähnte Formung der Federkörper 21 bis 21ₙ führt zu einer Federcharakteristik gemäß Fig. 14. Innerhalb eines Messbereiches zwischen einer Minimalkraft Fₘᵢₙ und einer Maximalkraft Fₘₐₓ verläuft die Formveränderung, also die Änderung der Höhe d (siehe Fig. 15) der Federkörper 21 bis 21ₙ zwischen einem Wert 1/dₘᵢₙ und einem Wert 1/dₘₐₓ im Wesentlichen linear. Je nach Anforderung, also je nach Messaufgabe werden die geometrische Form und Größe sowie das Material so bestimmt, dass Messungen in dem linearen Bereich gemäß Fig. 14 ermöglicht werden.

Eine Möglichkeit der Linearisierung von Federkörpern besteht darin, diese in Gruppen 21', 21"; 21_{1'}, 21_{1"}, 21_{2'}, 21_{2"} aufzuteilen, wobei die verschiedenen Körper 21' und 21"... geometrisch unterschiedlich geformt sind und unterschiedliche Federcharakteristiken (also Verhältnis d/F) aufweisen, wie dies in Fig. 16 gezeigt ist. Der in Fig. 15 gezeigte Federkörper 21' beruht auf Ausbeulung der Federkörperwände, so dass bei zunehmender Verformung die dafür notwendige Kraft abnimmt, während bei dem "massiven" Federkörper 21" die Kraft zunimmt. In der Summe kompensieren sich dadurch die beiden nichtlinearen Kurven. Natürlich ist es möglich, hier eine Vielzahl von anderen Kombinationen von Federkörpern zusammenwirken zu lassen.

### Bezugszeichenliste

- 10: Messfläche
- 11: Gegenfläche
- 12: Erstes Messelement/Elektrode
- 13: Zweites Messelement/Elektrode
- 14: Dielektrikum
- 15: Erstes Abschirmungselement
- 16: Zweites Abschirmungselement
- 20: Federeinrichtung
- 21 bis 21ₙ: Federkörper
- 22: Lücke
- 24: Trägerfläche

## Patentansprüche

1. Sensor zur elektrischen Messung einer auf den Sensor wirkenden Kraft (F) innerhalb eines vorgegebenen Messbereiches, die über eine Messfläche (10) des Sensors ungleichmäßig verteilt ist, wobei ein elektrisches Kraftmesssignal erzeugt wird, umfassend
eine flächig ausgebildete Federeinrichtung (20), die zwischen der Messfläche (10) und einer Gegenfläche (11) angeordnet ist,
wobei in oder auf der Messfläche (10) ein erstes Messelement und in oder auf der Gegenfläche (11) ein zweites Messelement, diese Flächen jeweils vollständig überdeckend angeordnet sind,
wobei die Messelemente Kondensatorplatten (12, 13) umfassen,
wobei die Messfläche (10) und die Gegenfläche (11) samt den Messelementen elastisch verformbar ausgebildet sind,
wobei die Messelemente mit den Kondensatorplatten (12, 13), derart ausgebildet sind, dass aus einem Abstand zwischen den Messelementen das Kraftmesssignal erzeugbar ist, wobei eine Kapazität zwischen den Kondensatorplatten zur Erzeugung des Kraftmesssignals messbar ist,
wobei die Federeinrichtung (20) eine Vielzahl von inkompressiblen aber elastisch ausgebildeten Federkörpern (21 bis 21ₙ) aufweist, die derart durch Lücken (22) voneinander beabstandet angeordnet sind, dass jeder Federkörper (21 bis 21ₙ) bei Belastung durch die Kraft (F) oder einen Bruchteil dieser Kraft (F) sich in die Lücken (22) hinein und somit raumgreifend verformen kann, und
wobei jeder Federkörper (21 bis 21ₙ) derart ausgebildet ist, dass seine Höhe zwischen der Messfläche (10) und der Gegenfläche (11) bei Belastung des Sensors durch die Kraft (F) innerhalb des vorgegebenen Messbereiches linear proportional zu einer auf ihn wirkenden Teilkraft ist,
**dadurch gekennzeichnet, dass**
die Messfläche (10) und die Gegenfläche (11) zusätzlich zum ersten bzw. zweiten Messelement auf ihren, der Federeinrichtung (20) abgewandten Seite Abschirmungselemente (15, 16) zur elektrischen Abschirmung der Messelemente umfassen, wobei die Kondensatorplatten (12, 13) und die Abschirmungen jeweils durch Dielektrika (14) voneinander getrennt sind, und
wobei eine Kapazitätsmesseinrichtung vorgesehen ist, die mit den Abschirmungselementen (15, 16) und den Kondensatorplatten (12, 13) verbunden und derart ausgebildet ist, dass Kapazitäten zwischen den Kondensatorplatten (12, 13) und den Abschirmungselementen (15, 16) zur Gewinnung eines Temperatursignals gemessen und das Kraftsignal entsprechend dem Temperatursignal mittels einer Korrektureinrichtung korrigierbar ist.

2. Sensor nach einem der vorhergehenden Ansprüche,
**dadurch gekennzeichnet, dass**
die Messfläche (10) und/oder die Gegenfläche (11) textiles Material, nämlich ein Gewirke oder ein Gewebe umfassen.

3. Sensor nach einem der vorhergehenden Ansprüche,
**dadurch gekennzeichnet, dass**
die Messfläche (10) und/oder die Gegenfläche (11) mit den Federkörpern (21 bis 21ₙ) durch Befestigungsmittel (24) verbunden sind.

4. Sensor nach Anspruch 3,
**dadurch gekennzeichnet, dass**
die Befestigungsmittel (24) elastische Verklebungen umfassen, die derart elastisch ausgebildet sind, dass die Messfläche (10) und/oder die Gegenfläche (11) relativ zur Federeinrichtung (20) verschiebbar ist.

5. Sensor nach einem der vorhergehenden Ansprüche,
**dadurch gekennzeichnet, dass**
die Federkörper (21 bis 21ₙ) in regelmäßigen Abständen voneinander angeordnet sind, wobei die Federkörper (21 bis 21ₙ) aus Silikonkautschuk mit einer Shore-Härte von 45-55gefertigt sind.

6. Sensor nach Anspruch 5,
**dadurch gekennzeichnet, dass**
die Federkörper (21 bis 21ₙ) als balkenförmige, pyramidenstumpfförmige oder kegelstumpfförmige Einzelelemente ausgebildet sind.

7. Sensor nach einem der vorhergehenden Ansprüche,
**dadurch gekennzeichnet, dass**
die Federkörper (21 bis 21ₙ) über mindestens eine Trägerfläche (24) miteinander verbunden sind.

8. Sensor nach Anspruch 7,
**dadurch gekennzeichnet, dass**
die Trägerfläche (24) mit den Federkörpern (21 bis 21ₙ) einstückig ausgebildet ist.

9. Verwendung eines Sensors nach einem der vorhergehenden Ansprüche zur Messung von Belastungen, welche menschliche Gliedmaßen oder Effektoren eines Roboters auf eine Umgebung ausüben.

10. Verwendung eines Sensors nach Anspruch 9,
**dadurch gekennzeichnet, dass**
die Umgebung ein Schuh, eine Prothese, eine Orthese, ein Griff, ein Lenkrad, ein natürliches oder ein künstliches Gelenk, ein Werkzeug oder ein Werkstück ist.

## Claims

1. A sensor for electrically measuring a force (F) acting upon the sensor within a predefined measuring range, and which is unevenly distributed across a measuring surface (10) of the sensor, wherein an electrical force measurement signal is generated, comprising
a spring device (20) of a two-dimensional configuration, which is arranged between the measuring surface (10) and a opposite surface (11),
wherein in or on the measuring surface (10) a first measuring element and in or on the opposite surface (11) a second measuring element are arranged each covering completely said faces,
wherein the measuring elements comprise capacitor plates (12, 13),
wherein the measuring surface (10) and the opposite surface (11) together with the measuring elements are formed to be elastically deformable,
wherein the measuring elements having the capacitor plates (12, 13) are formed such that a force measurement signal can be generated from a distance between the measuring elements, wherein a capacity between the capacitor plates is measurable for generating the force measurement signal,
wherein the spring device (20) includes a plurality of incompressible but elastically formed spring bodies (21 to 21ₙ) which are arranged to be spaced from each other by gaps (22) such that each of the spring bodies (21 to 21ₙ), when being loaded by the force (F) or a fraction of said force (F), may deform into the gaps (22) and thus may deform in a space-occupying manner, and
wherein each of the spring bodies (21 to 21ₙ) is formed such that its height between the measuring surface (10) and the opposite surface (11), when the sensor is being loaded by the force (F) within the predefined measuring range, is linearly proportional to a partial force acting upon it,
**characterized in that**
the measuring surface (10) and the opposite surface (11), in addition to the first and second measuring element, respectively include, on the side facing away from the spring device (20), shielding elements (15, 16) for electrically shielding the measuring elements, wherein the capacitor plates (12, 13) and the shielding elements each are separated from each other by dielectrics (14), and
wherein a capacity measuring device is provided, which is connected with the shielding elements (15, 16) and the capacitor plates (12, 13) and is formed such that capacities between the capacitor plates (12, 13) and the shielding elements (15, 16) can be measured for obtaining a temperature signal, and the force signal can be corrected corresponding to the temperatures signal by means of a correcting device.

2. The sensor according to any one of the preceding claims,
**characterized in that**
the measuring surface (10) and/or the opposite surface (11) comprise a textile material, particularly a non-woven fabric or a woven fabric.

3. The sensor according to any one of the preceding claims,
**characterized in that**
the measuring surface (10) and/or the opposite surface (11) are connected with the spring bodies (21 to 21ₙ) by fastening means (24).

4. The sensor according to claim 3,
**characterized in that**
the fastening means (24) comprise elastic adhesive bonds which are formed to be elastic in such a manner that the measuring surface (10) and/or the opposite surface (11) is/are movable in relation to the spring device (20).

5. The sensor according to any one of the preceding claims,
**characterized in that**
the spring bodies (21 to 21ₙ) are arranged at regular intervals from each other, wherein the spring bodies (21 to 21ₙ) are manufactured from silicone rubber having a Shore hardness of 45 to 55.

6. The sensor according to claim 5,
**characterized in that**
the spring bodies (21 to 21ₙ) are formed as beam-shaped, truncated pyramid-shaped or truncated cone-shaped single elements.

7. The sensor according to any one of the preceding claims,
**characterized in that**
the spring bodies (21 to 21ₙ) are interconnected by at least one carrier surface (24).

8. The sensor according to claim 7,
**characterized in that**
the carrier surface (24) is formed integrally with the spring bodies (21 to 21ₙ).

9. Use of a sensor according to any one of the preceding claims for measuring loads exerted upon an environment by human limbs or effectors of a robot.

10. Use of a sensor according to claim 9,
**characterized in that**
the environment is a shoe, a prosthesis, an orthosis, a handle, a steering wheel, a natural or an artificial joint, a tool or a workpiece.

## Revendications

1. Capteur pour la mesure électrique d'une force (F) agissant sur le capteur au sein d'une plage de mesure spécifiée et répartie inégalement sur une face de mesure (10) du capteur, sachant qu'un signal électrique de mesure de force est généré, comprenant
un dispositif ressort (20) constitué de manière plane, lequel est disposé entre la face de mesure (10) et une face opposée (11),
sachant qu'un premier élément de mesure est disposé dans ou sur la face de mesure (10) et un deuxième élément de mesure est disposé dans ou sur la face opposée (11) en recouvrant respectivement entièrement ces faces,
sachant que les éléments de mesure comprennent des armatures de condensateur (12, 13),
sachant que la face de mesure (10) et la face opposée (11) avec les éléments de mesure sont constituées de manière élastiquement déformable,
sachant que les éléments de mesure avec les armatures de condensateur (12, 13) sont constituées de telle manière que le signal de mesure de force puisse être généré à partir d'une distance entre les éléments de mesure, sachant qu'une capacité entre les armatures de condensateur est mesurable pour la génération du signal de mesure de force,
sachant que le dispositif ressort (20) présente une pluralité de corps de ressort (21 à 21ₙ) incompressibles mais constitués de manière élastique, lesquels sont disposés de manière espacée les uns des autres par des interstices (22) de telle manière que chaque corps de ressort (21 à 21ₙ) puisse se déformer en cas de sollicitation par la force (F) ou une fraction de cette force (F) en entrant dans les interstices (22) et en prenant ainsi de l'espace, et
sachant que chaque corps de ressort (21 à 21ₙ) est constitué de telle manière que sa hauteur entre la face de mesure (10) et la face opposée (11), lors d'une sollicitation du capteur par la force (F) au sein de la plage de mesure spécifiée, soit linéairement proportionnelle à une force partielle agissant sur lui,
**caractérisé en ce que**
la face de mesure (10) et la face opposée (11) comprennent, en plus du premier ou deuxième élément de mesure, de leur côté opposé au dispositif ressort, des éléments de blindage (15, 16) pour le blindage électrique des éléments de mesure, sachant que les armatures de condensateur (12, 13) et les blindages sont respectivement séparés les uns des autres par des diélectriques (14), et
sachant qu'un dispositif de mesure de capacité est prévu, lequel est relié aux éléments de blindage (15, 16) et aux armatures de condensateur (12, 13) et est constitué de telle manière que des capacités entre les armatures de condensateur (12, 13) et les éléments de blindage (15, 16) soient mesurées pour l'obtention d'un signal de température et le signal de force puisse être corrigé selon le signal de température moyennant un dispositif de correction.

2. Capteur selon l'une des revendications précédentes,
**caractérisé en ce que**
la face de mesure (10) et/ou la face opposée (11) comprennent de la matière textile, à savoir un tricoté ou une étoffe.

3. Capteur selon l'une des revendications précédentes,
**caractérisé en ce que**
la face de mesure (10) et/ou la face opposée (11) sont reliées aux corps de ressort (21 à 21ₙ) par des moyens de fixation (24).

4. Capteur selon la revendication 3,
**caractérisé en ce que**
les moyens de fixation (24) comprennent des collages élastiques qui sont constitués de manière élastique de telle manière que la face de mesure (10) et/ou la face opposée (11) soit déplaçable / soient déplaçables relativement au dispositif ressort (20).

5. Capteur selon l'une des revendications précédentes,
**caractérisé en ce que**
les corps de ressort (21 à 21ₙ) sont disposés à distances régulières les uns des autres, sachant que les corps de ressort (21 à 21ₙ) sont fabriqués à partir de caoutchouc de silicone d'une dureté Shore de 45 à 55.

6. Capteur selon la revendication 5,
**caractérisé en ce que**
les corps de ressort (21 à 21ₙ) sont constitués comme éléments individuels en forme de barreaux, de pyramides tronquées ou de cônes tronqués.

7. Capteur selon l'une des revendications précédentes,
**caractérisé en ce que**
les corps de ressort (21 à 21ₙ) sont reliés les uns aux autres par au moins une face support (24).

8. Capteur selon la revendication 7,
**caractérisé en ce que**
la face support (24) est constituée d'une seule pièce avec les corps de ressort (21 à 21n).

9. Utilisation d'un capteur selon l'une des revendications précédentes pour la mesure de sollicitations que des membres humains ou des effecteurs d'un robot exercent sur un environnement.

10. Utilisation d'un capteur selon la revendication 9,
**caractérisé en ce que**
l'environnement est une chaussure, une prothèse, une orthèse, une poignée, un volant, une articulation naturelle ou artificielle, un outil ou une pièce à travailler.
